# EUROPEAN PATENT SPECIFICATION

(11) **EP 1 800 609 B1**
(45) Date of publication and mention of the grant of the patent: **24.11.2010**
(21) Application number: 06026288.8
(22) Date of filing: 19.12.2006
(51) Int. Cl.: A61B 17/06, A61L 17/00, A61L 17/14

(54) **System and method for coating filaments**
System und Verfahren zum Ummanteln von Fäden
Système et méthode pour revêtir des filaments

(30) Priority: 20.12.2005 US 752685 P
(43) Date of publication of application: 27.06.2007
(60) Divisional application: 10003090.7
(73) Proprietor: Tyco Healthcare Group LP, Norwalk, CT 06856 (US)
(72) Inventor: Tsai, Steven, Stamford, CT 06905 (US)
(74) Representative: Soames, Candida Jane

(56) References cited:
- EP-A1- 1 074 270
- EP-A2- 0 839 542
- WO-A-86/00020
- US-A1- 4 911 927
- US-A1- 5 104 398
- US-A1- 5 312 642

## Description

### BACKGROUND

### 1. Technical Field

The present disclosure relates generally to filament coating systems and methods, more specifically to systems and methods for coating sutures.

### 2. Background of Related Art

Surgical sutures are primarily used during surgery to stitch together sections of tissue to aid in post-surgical healing. Sutures are often coated with various substances to improve their knot tie-down characteristics. In addition, coating increases suture's surface lubricity which reduces the friction associated with passing of the suture through the tissue, thereby reducing tissue trauma. Conventionally, suture coatings have been applied by dipping, bushing, wiping, spraying or drip coating. Dip coating involves submergence of a suture line into a coating composition contained in a vessel. Typically, dip coating involves the use of multiple guiding means (e.g., rollers) to pass the suture through the coating composition. For instance, a horizontally passing suture needs to be directed downward into and upward out of the vessel containing the coating composition. Thus, at least one of the guiding roller(s) is positioned within the coating vessel, in contact with the suture before the line is dried. This contact by the roller frequently removes coating material from the suture, thereby reducing the effectiveness of the coating.

Coating has also been accomplished using filling heads. This method involves passing a suture line through a V-shaped notch. Coating composition injected into the notch contacts and coats the suture line. The suture line does not come in contact with guiding rollers until after the coating dries. Although the coating system using filling heads provides more consistent coating for the suture line, the contact time for coating solution to penetrate into suture is significantly less (e.g., less that 0.1 second) than that provided by the conventional dipping coating mechanisms.

EP 1 074 270 discloses a melt coating die assembly for coating a surgical filament.

US 4,911,927 discloses a bath for adding chemotherapeutic agents to dental floss. The pre-amble of the independent claims is based on this document.

US 5,104,398 discloses an impregnating tank containing a solution for coating a surgical sutured thread.

Therefore, there is a need for a coating system which allows for more consistent coating at high contact time.

### SUMMARY

A system and method for coating a suture line are disclosed. The system includes an elongated coating tube having coater heads disposed at both ends thereof. The suture line is fed into the coating tube through an opening in one of the coater heads and exits the coating tube through an opening in the other coater head. Guide rollers that contact the un-coated suture ensure alignment of the suture line as it enters the coating tube. The coating tube is filled with a coating composition to a level above the entry point of the suture line. The composition pours through the openings of the coating tube and is collected by the coater heads so that it is circulated during the coating process-to ensure proper ratio of coating agent to solvent. After exiting the coating tube, the suture line passes through a dryer and then is guided by a set of rollers to a winding roll. Then, the suture contacts guide rollers only after exiting the dryer, thereby helping to ensure a uniform coating.

According to an embodiment of the present disclosure, a system for coating at least one suture line is disclosed. The system includes a first guide positioned to orient the suture line for entry into a coating tube having an inlet opening to admit the suture line passing through the first guide and an outlet opening through which the suture line exits the coating tube. The coating tube also includes at least one fill opening through which a coating composition is introduced into the coating tube to at least a level sufficient to submerge at least one portion of the suture line, such that the coating composition flows out through at least one of the inlet and outlet openings. The system also includes at least one coater head disposed at each of end of the coating tube, the first and second coater head configured to collect the coating composition flowing through the inlet and outlet openings and a second guide positioned to orient the suture line exiting the coating tube.

According to another embodiment of the present disclosure, a method for coating at least one suture line is disclosed, using the coating system previously mentioned.

### BRIEF DESCRIPTION OF THE DRAWINGS

The above and other aspects, features, and advantages of the present disclosure will become more apparent in light of the following detailed description when taken in conjunction with the accompanying drawings in which:
Fig. 1 is a schematic diagram of a dip suture coating system according to one embodiment of the present disclosure;
Fig. 2 is a schematic diagram of a dip coater according to the present disclosure;
Fig. 3 is a schematic diagram of a distal coater head useful in embodiments of coating systems according to the present disclosure;
Fig. 4 is a sectional view of a dip coating tube according to the present disclosure; and
Fig. 5 is a flow diagram of a method for coating a suture line according to the present disclosure.

### DETAILED DESCRIPTION

Preferred embodiments of the present disclosure will be described herein below with reference to the accompanying drawings. In the following description, well-known functions or constructions are not described in detail to avoid obscuring the present disclosure in unnecessary detail.

The present disclosure provides for a horizontal dip coating system. The system includes one or more input winders inputting a suture line into a dip coater using a first set of one or more guiding rollers. The dip coater includes a coating tube positioned horizontally with a coater head disposed at proximal and distal ends thereof. The coating tube and the coater heads include openings through its centers to allow for the suture line to pass horizontally therethrough. A coating composition fills the coating tube above the openings of the coating tube through which the suture line passes to coat the line. Any coating composition that leaks out of the coating tube is collected by the coater heads. The coating composition is injected at a higher rate than it leaks out to maintain the composition level therein. In addition, the coating composition collected by the coater heads is circulated throughout the dip coater. In some embodiments, where the coating composition includes solvent carrier and coating agent, solvent content of the coating composition is monitored and adjusted to ensure that a predetermined ratio of coating agent to solvent is maintained. Upon coating, the suture line is dried and thereafter the line is guided by a second set of one or more rollers to winding rolls.

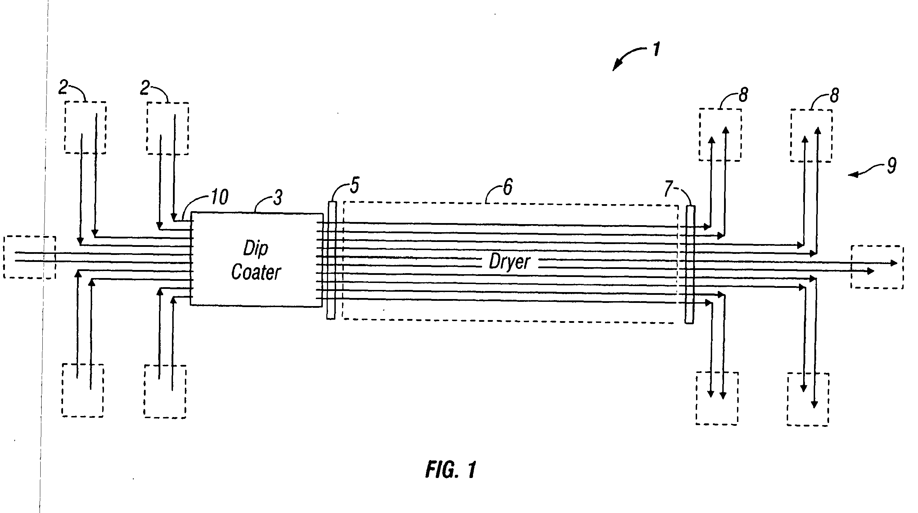

Fig. 1 shows a horizontal dip coating system 1 according to the present disclosure for coating a plurality of suture lines simultaneously. The coating system 1 includes at least one input winder 2 for passing at least one suture line 10 through a dip coater 3, an air wiper 5, a dryer 6, and an air cooler 7. Thereafter the line 10 is wound by at least one take-up winder 8, which may be part of a pull-over loading station 9. It is envisioned that the pull-over loading station pulls the line 10 through the coating system 1. As the line 10 passes through the dip coater 3 it is submerged in a coating composition in order to absorb coating materials. As will be appreciated by those skilled in the art, the coating system shown in Fig. 1 includes multiple input winders and multiple take-up winders. Also, it is contemplated that each input winder and/or take-up winder may handle one or more than one suture lines (e.g., in Fig. 1 each winder handles two suture lines).

The input winder(s) 2 disperses the line 10 which may be a monofilament or a multifilament braided suture. Prior to dispersing, the line 10 may be prepared for coating which may involve calendaring the line 10 to facilitate penetration of the coating composition into the interstices of the braid suture, especially where the present system is used to apply a second or third composition to the suture line. An example of a calendaring apparatus and method thereof is disclosed by commonly owned U.S. Patent No. 5,312,642 entitled "Method and Apparatus for Calendering and Coating/Filling Sutures".

The system 1 also includes the air wiper 5 and the air cooler 7. The air wiper 5 is positioned between the dip coater 3 and the dryer 6 and is configured to blow gas (e.g., air, nitrogen, etc.) on the passing line 10 to blow off excess composition. The air cooler 7 is positioned between the dryer 6 and the take-up winder 8 and is configured to blow cool air on the line 10 to provide cooling for the dried line.

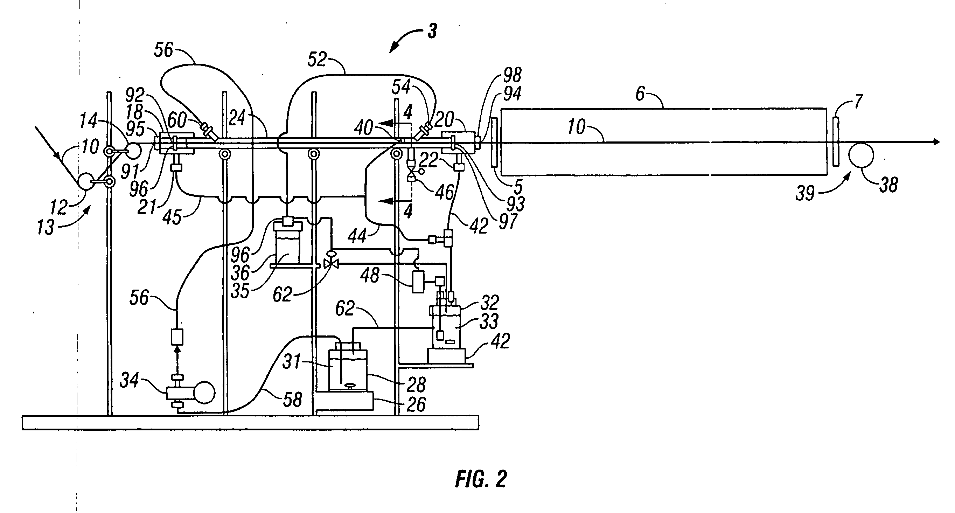

Fig 2 shows in more detail a dip coater 3 and the dryer 6 in accordance with an embodiment of the present disclosure. The line 10 is passed through the dip coater 3 so that it is immersed in a coating composition. The line 10 is guided into the dip coater 3 around a first set of rollers 13 (e.g., rollers 12 and 14) and passed horizontally through a coating tube 24 which is filled with a coating composition 31 containing the dissolved coating agent. It is envisioned that the dip coater 3 may be configured to coat a plurality of lines 10. For example, the dip coater 3 may have a plurality of coating tubes 24 corresponding to the number of lines 10. Alternatively, the coating tube 4 may be substituted by a container configured to coat multiple lines 10.

As the line 10 is passed horizontally through the coating tube 24, coating composition is applied to the line 10. The coating tube 24 is positioned horizontally and may have a cylindrical shape and may be approximately 1' to 10' (0.3m to 3m) in length. Typically, the coating tube is 3' to 5' (0.9m to 2m) long. In addition, the coating tube 24 is preferably manufactured from stainless steel material that does not react with the coating composition. For example, the coating tube may be glass or glass-lined. In addition, if a metal tube is used, it may be desirable to passivate the tube to reduce its reactivity. Passivation methods and materials are within the purview of those skilled in the art. The outer diameter of tube can vary depending on a number of factors such as the number of filaments being treated, the particular coating composition being applied and the desired contact time between the composition and the suture line. Those skilled in the art will appreciate that the cylindrical shape is merely only one embodiment of the coating tube 24 and that it may have a variety of shapes (e.g., rectangular, triangular, or hexagonal cross-sectional shapes, etc.).

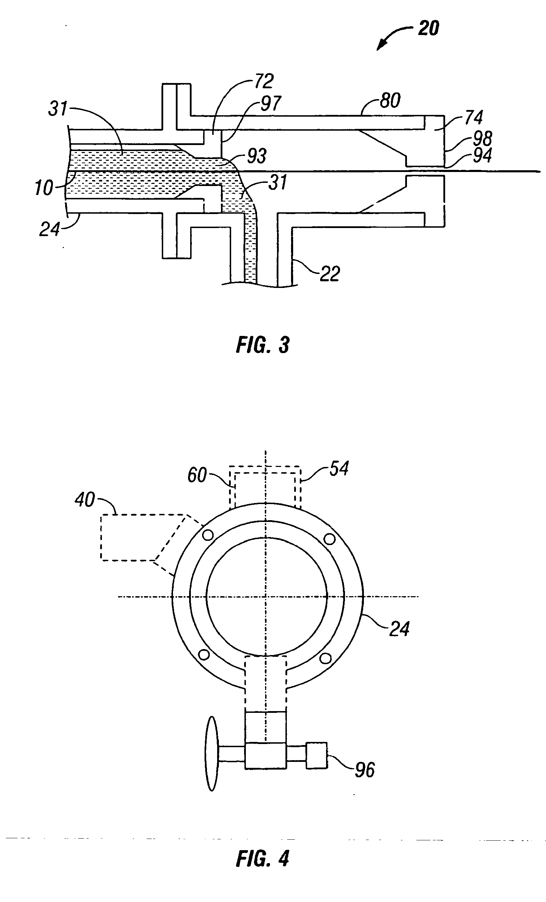

The line 10 enters the coating tube 24 through a proximal coater head 18 which is discussed in more detail below with reference to a distal coater head 20, both of which have a circular cross section. It is envisioned that the line 10 passes through dip coater 3 through an opening 91 of the proximal coater head 18, an opening 92 of the coating tube 24, an opening 93 of the coating tube 24 and an opening 94 of the distal coater head 20. The openings 91, 92, 93, 94 are preferably drilled through the center of surfaces 95, 96, 97, 98 respectively, of ceramic eyelets disposed within the proximal and distal coater heads 18, 20 and the coating tube 24 as shown in Fig. 3 and discussed in more detail below. The openings may be drilled in a plurality of ways (e.g., a laser) to achieve a straight path for the line 10 through the dip coater 3.

The coating tube 24 is filled with a coating composition 31 through a supply tube 56 which is connected thereto via adapter 60 having a male national pipe thread (MNPT) or another type of connecting means. The adapter 60 may be coupled to the coating tube 24 at an angle (e.g., a 135° angle) with the longitudinal axis of the coating tube 24 at the top portion thereof. Those skilled in the art will appreciate that the tubing used to interconnect the components of the dip coater 3 (e.g., the supply tube 56 and other tubing discussed below) may be manufactured from any materials allowing the tubing to be flexible as well as chemically inert to a variety of organic solvents. In one embodiment, the supply tube 56 is made from PTFE and has a 3/8" (10mm) diameter. The coating composition 31 is carried into the coating tube 24 via a circulating pump 34 which withdraws the composition 31 from a tank 28 through a tube 58. The tank 28 preferably includes a stirring device, such as a magnetic stirrer driver 26 positioned thereunder, to prevent settling or other separation of the components of the coating composition.

Fig. 3 shows a cross sectional view of the distal coater head 20 along the vertical axis of the coating tube 24. The coating tube 24 is filled with the composition 31 to a level which is at least above the line 10 (e.g., above the center of the coating tube 24) to ensure that the line 10 is immersed in the composition 31 as the line 10 passes through the coating tube 24. Since the composition 31 fills the coating tube 24 above the opening 93, composition 31 may leak therethrough and be collected in the proximal and distal coater heads 18, 20. As a result of composition 31 leaking out of the coating tube 24, the in-flow must equal or exceed outflow. Therefore, the composition 31 will be circulated (e.g., continually supplied into the coating tube 24) to maintain a steady state, where the level of the composition 31 is continuously above the openings 92, 93.

The assembly 20 includes a removable reducing tee 80 configured to drain leaking composition 31. The reducing tee 80 can advantageously have an outer diameter larger than the outer diameter of the coating tube 24 so that the tee 80 fits over the coating tube 24. The return adapter 22 is the reducing portion of the reducing tee 80 and preferably includes an MNPT connection.

The removable tee 80 is connected to the coating tube 24. The distal coater head 20 includes two ceramic eyelets 72, 74. The ceramic eyelet 72 is positioned at the distal end of the coating tube 24 within the reducing tee 80 and the ceramic eyelet 74 is attached at the distal end of the reducing tee 80. The ceramic eyelets 72, 74 are designed to optimize alignment of the openings 93, 94 while minimizing the solvent lost during the coating process as discussed in more detail below. Those skilled in the art will appreciate that the proximal coater head 18 includes the same components (e.g., ceramic eyelet, o-ring, etc.) assembled in the same manner as discussed above with regard to the distal coater head 20.

With reference to Fig. 2, the composition 31 out-flows through return adapters 21, 22 coupled to the bottom portion of the proximal and distal coater heads 18, 20 respectively and return tubes 45, 42 to a return tank 32. In addition, the return tank 32 collects any excess composition 31 present in the coating tube 24 through an overflow adapter 40 which is coupled to the upper portion thereof. Upon reaching the overflow adapter 40, the excess coating composition 31 flows through a return tube 44 to the return tank 32 and mixes with return composition 33. The outlet of overflow adapter 40 is also used to determine when the composition 31 filled the coating tube 24 to the desired level. The overflow adapter 40 is positioned above the openings 91, 92, 93, 94 hence when composition 31 reaches the overflow adapter 40 and flows therethrough and into the return tank 32. This ensures that the suture line 10 is submerged inside the coating tube 24. There can be multiple overflow adapters 40 positioned along the coating tube 24 for higher recirculation rate.

Fig. 4 shows a cross sectional view along the section line 4 of the coating tube 24. Adapter 60 and adapter 54 are shown positioned on the upper portion of the coating tube 24 while the drain valve 46 is positioned on the bottom portion thereof. In addition, the overflow adapter 40 is positioned on the upper portion of the coating tube 24 as well, slightly below the adapters 54, 60. The drain valve 46 may be used to empty coating tube 24 of the coating composition 31 upon shut down of the coating system 1 for maintenance or once the coating process is finished.

It is also envisioned that the return composition 33 may be recycled during the coating process. Typically, a portion of any volatile solvent in the coating composition may evaporate during the coating process before excess composition 31 flows to the return tank 32. The return composition 33 is contained within the coating system 1 by a plurality of tubes and adapters discussed above to contain the return composition 33 and to minimize solvent loss and vapor escape. Some vapor which does escape is vented through vent headers (not shown) installed above the coating tube 24.

In order for the return composition 33 to be recycled, it must be verified that the desired coating agent composition is maintained. With reference to Fig. 2, a controller 48 can be connected to the return tank 32 and configured to test the density and/or viscosity of the return composition 33 using, e.g., a mass flow meter. If the density/viscosity of the return composition 33 exceeds a predetermined level (e.g., density of the original composition 31) then the controller 48 signals that the composition needs to be adjusted e.g., by injecting additional solvent carrier 35 directly into the coating tube 24 or into the return tank 32.

In one embodiment, the controller 48 is electrically connected to a solvent pump 96 (e.g., solvent supply means) which injects solvent 35 into the coating tube 24 and a control valve 50 adjusts the flow of the solvent 35 into the return tank 32. The solvent 35 can be injected into the coating tube 24 through the solvent adapter 54 connected to the solvent tank 36 through a tube 52. The solvent adapter 54 is positioned at an angle (e.g., a 45° angle) with the longitudinal axis of the coating tube 24 at the top portion thereof while the drain valve 46 is positioned on the bottom portion thereof. In addition, the return tank 32 includes a magnetic stirrer driver 30 to maintain uniformity of the coating composition throughout the return composition 33 and to mix any added solvent 35. Furthermore, return tank 32 supplies the return composition 33 to tank 28 where it is recycled and continually pumped into the coating tube 24.

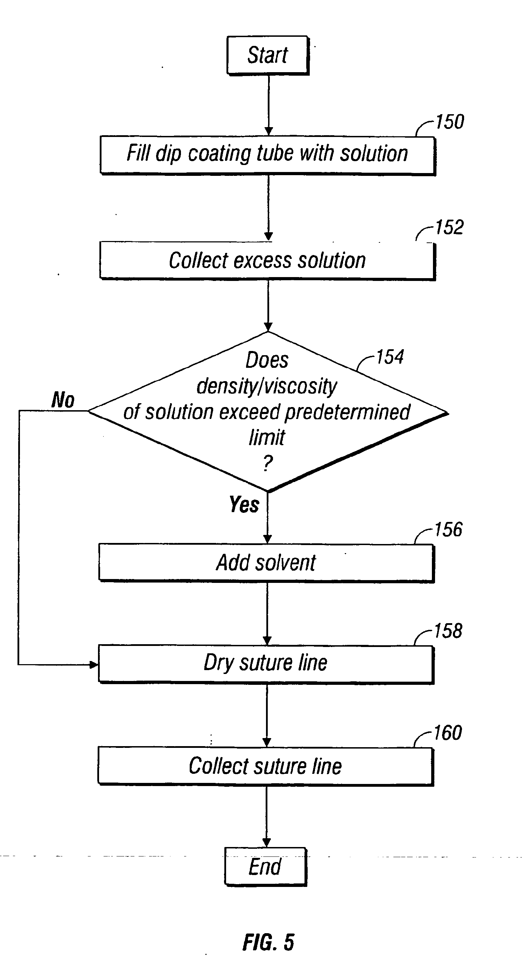

Fig. 5 shows a method for coating the suture line 10 using the coating system 1 of the present disclosure. In step 150 the coating tube 24 is filled with the composition 31 above the openings 92, 93 to ensure that as the line 10 is submerged in the composition 31. The composition 31 is continually fed into the coating tube 24 at a rate which is equal to or slightly exceeds the rate at which the composition 31 leaks through the openings 92, 93. This allows the level of the composition 31 to stay above the openings 92, 93 despite the loss of the composition 31. Thus, only the level of the composition 31 must be controlled during the coating process, whereas, conventional coating techniques, which depend on point coating, require precise control of the flow rate of the coating composition.

In step 152, excess composition 31 is collected. This is accomplished by collecting the composition 31 from the proximal and distal coater heads 18, 20 which are configured to catch excess liquid and from the overflow adapter 40 which prevents overfilling of the coating tube 24 by providing a flow path for any excess composition 31. The excess composition 31 flows to the return tank 32.

Since the coating system 1 relies on constant supply of the composition 31 to the coating tube 24 the composition 31 must be circulated. As discussed above, in some embodiments the coating process involves application of coating agent dissolved in a volatile organic solvent. Therefore, solvent may need to be replenished to maintain the desired coating agent levels within the composition 31. In step 154, the controller 48 analyzes the density/viscosity of return composition 33 to determine whether additional solvent 35 needs to be added. If the density is above the desired limit (e.g., the coating agent to solvent ratio within the return composition 33 is larger than that ratio within the composition 31) then in step 156, the controller 48 adjusts the solvent content. Adjustment of solvent level can be accomplished by signaling the solvent pump 96 which injects the solvent 35 into the coating tube 24 and/or opening the control valve 50 to add solvent 35 into the return composition 33. The return composition 33 is then added into tank 28 from where it is continuously injected into coating tube 24.

Each portion of the suture line 10 should reside within the coating tube 24 approximately between 0.5 second to 10 minutes, preferably the residence time is about 1 minute. As the line 10 the dip coater 3 the line 10 enters the dryer 6 and in step 158, the line 10 is dried. The drying temperature depends on the coating composition used and may range approximately between the ambient room temperature (e.g., 25°C) at the low extreme and above 100°C at the upper extreme. It is also envisioned that the dryer 6 uses heated gas (e.g., air, nitrogen, etc.) to dry the line 10. In step 160, the line 10 is cooled by the air cooler 7, which blows cool air onto the dried line 10 and thereafter the line 10 is guided by a second set of rollers 39 (e.g., a roller 38) into the winder 8 where it is wound.

The present disclosure allows the line 10 to avoid physical contact with various machine parts during the coating process (e.g., guiding rollers). The line 10 comes in contact only with the coating composition between the time it is fed into the dip coater 3 and the time it is wound on the winder 8. This is due to the horizontal positioning of the coating tube 24 and the horizontal orientation of the line 10 therethrough, which allows the line 10 to avoid physical contact with components by using a minimal amount of guiding mechanisms (e.g., guiding rollers 13, 39)

Any coating composition known to be useful for coating medical devices may be applied to a medical device using the present methods and apparatus. The coating composition can be a solution, dispersion, emulsion containing, for example, one or more polymeric materials and/or one or more bioactive agents.

In some embodiments, the coating composition includes a polymer, or a combination of polymers. The polymer is most suitably biocompatible, including polymers that are nontoxic, non-inflammatory, chemically inert, and substantially non-immunogenic in the applied amounts. The polymer may be either bioabsorbable or biostable. A bioabsorbable polymer breaks down in the body. Bioabsorbable polymers are gradually absorbed or eliminated by the body by hydrolysis, metabolic process, bulk, or surface erosion. Examples of bioabsorbable materials include but are not limited to polycaprolactone (PCL), poly-D, L-lactic acid (DL-PLA), poly-L-lactic acid (L-PLA), poly(lactide-co-glycolide), poly(hydroxybutyrate), poly(hydroxybutyrate-co-valerate), polydioxanone, polyorthoester, polyanhydride, poly(glycolic acid), poly(glycolic acid-cotrimethylene carbonate), polyphosphoester, polyphosphoester urethane, poly (amino acids), cyanoacrylates, poly(trimethylene carbonate), poly(iminocarbonate), copoly(ether-esters), polyalkylene oxalates, polyphosphazenes, polyiminocarbonates, and aliphatic polycarbonates. Biomolecules such as heparin, fibrin, fibrinogen, cellulose, starch, and collagen are typically also suitable. A biostable polymer does not break down in the body, and thus a biostable polymer is present in the body for a substantial amount of time after implantation. Examples of biostable polymers include PARYLENE^{™}, PARYLAST^{™}, polyurethane (for example, segmented polyurethanes such as BIOSPAN™), polyethylene, polyethlyene teraphthalate, ethylene vinyl acetate, silicone, polyethylene oxide, and polytetrafluoroethylene (PTFE).

In some embodiments, the coating compositions of the present disclosure may also include a fatty acid component that contains a fatty acid or a fatty acid salt or a salt of a fatty acid ester. Suitable fatty acids may be saturated or unsaturated, and include higher fatty acids having more than about 12 carbon atoms. Suitable saturated fatty acids include, for example, stearic acid, palmitic acid, myristic acid and lauric acid. Suitable unsaturated fatty acids include oleic acid, linoleic acid, and linolenic acid. In addition, an ester of fatty acids, such as sorbitan tristearate or hydrogenated castor oil, may be used.

Suitable fatty acid salts include the polyvalent metal ion salts of C6 and higher fatty acids, particularly those having from about 12 to 22 carbon atoms, and mixtures thereof. Fatty acid salts including the calcium, magnesium, barium, aluminum, and zinc salts of stearic, palmitic and oleic acids may be useful in some embodiments of the present disclosure. Particularly useful salts include commercial "food grade" calcium stearate which consists of a mixture of about one-third C16 and two-thirds C18 fatty acids, with small amounts of the C 14 and C22 fatty acids.

Suitable salts of fatty acid esters which may be included in the coating compositions applied in accordance with the present disclosure include calcium, magnesium, aluminum, barium, or zinc stearoyl lactylate; calcium, magnesium, aluminum, barium, or zinc palmityl lactylate; calcium, magnesium, aluminum, barium, or zinc olelyl lactylate; with calcium stearoyl-2-lactylate (such as the calcium stearoyl-2-lactylate commercially available under the tradename VERV from American Ingredients Co., Kansas City, Mo.) being particularly useful. Other fatty acid ester salts which may be utilized include those selected from the group consisting of lithium stearoyl lactylate, potassium stearoyl lactylate, rubidium stearoyl lactylate, cesium stearoyl lactylate, francium stearoyl lactylate, sodium palmityl lactylate, lithium palmityl lactylate, potassium palmityl lactylate, rubidium palmityl lactylate, cesium palmityl lactylate, francium palmityl lactylate, sodium olelyl lactylate, lithium olelyl lactylate, potassium olelyl lactylate, rubidium olelyl lactylate, cesium olelyl lactylate, and francium olelyl lactylate.

Where utilized, the amount of fatty acid component can range in an amount from about 5 percent to about 50 percent by weight of the total coating composition. Typically, the fatty acid component may be present in an amount from about 10 percent to about 20 percent by weight of the total coating compositions.

In some embodiments, the coating composition contains one or more bioactive agents. The term "bioactive agent", as used herein, is used in its broadest sense and includes any substance or mixture of substances that have clinical use. Consequently, bioactive agents may or may not have pharmacological activity per se, e.g., a dye. Alternatively a bioactive agent could be any agent which provides a therapeutic or prophylactic effect, a compound that affects or participates in tissue growth, cell growth, cell differentiation, a compound that may be able to invoke a biological action such as an immune response, or could play any other role in one or more biological processes.

Examples of classes of bioactive agents which may be utilized in accordance with the present disclosure include antimicrobials, analgesics, antipyretics, anesthetics, antiepileptics, antihistamines, anti-inflammatories, cardiovascular drugs, diagnostic agents, sympathomimetics, cholinomimetics, antimuscarinics, antispasmodics, hormones, growth factors, muscle relaxants, adrenergic neuron blockers, antineoplastics, immunogenic agents, immunosuppressants, gastrointestinal drugs, diuretics, steroids, lipids, lipopolysaccharides, polysaccharides, and enzymes. It is also intended that combinations of bioactive agents may be used.

Suitable antimicrobial agents which may be included as a bioactive agent in the bioactive coating of the present disclosure include triclosan, also known as 2,4,4'-trichloro-2'-hydroxydiphenyl ether, chlorhexidine and its salts, including chlorhexidine acetate, chlorhexidine gluconate, chlorhexidine hydrochloride, and chlorhexidine sulfate, silver and its salts, including silver acetate, silver benzoate, silver carbonate, silver citrate, silver iodate, silver iodide, silver lactate, silver laurate, silver nitrate, silver oxide, silver palmitate, silver protein, and silver sulfadiazine, polymyxin, tetracycline, aminoglycosides, such as tobramycin and gentamicin, rifampicin, bacitracin, neomycin, chloramphenicol, miconazole, quinolones such as oxolinic acid, norfloxacin, nalidixic acid, pefloxacin, enoxacin and ciprofloxacin, penicillins such as oxacillin and pipracil, nonoxynol 9, fusidic acid, cephalosporins, and combinations thereof. In addition, antimicrobial proteins and peptides such as bovine lactoferrin and lactoferricin B may be included as a bioactive agent in the bioactive coating of the present disclosure.

Other bioactive agents which may be included as a bioactive agent in the coating composition applied in accordance with the present disclosure include: local anesthetics; non-steroidal antifertility agents; parasympathomimetic agents; psychotherapeutic agents; tranquilizers; decongestants; sedative hypnotics; steroids; sulfonamides; sympathomimetic agents; vaccines; vitamins; antimalarials; anti-migraine agents; anti-parkinson agents such as L-dopa; anti-spasmodics; anticholinergic agents (e.g. oxybutynin); antitussives; bronchodilators; cardiovascular agents such as coronary vasodilators and nitroglycerin; alkaloids; analgesics; narcotics such as codeine, dihydrocodeinone, meperidine, morphine and the like; non-narcoties such as salicylates, aspirin, acetaminophen, d-propoxyphene and the like; opioid receptor antagonists, such as naltrexone and naloxone; anti-cancer agents; anti-convulsants; anti-emetics; antihistamines; anti-inflammatory agents such as hormonal agents, hydrocortisone, prednisolone, prednisone, non-hormonal agents, allopurinol, indomethacin, phenylbutazone and the like; prostaglandins and cytotoxic drugs; estrogens; antibacterials; antibiotics; anti-fungals; anti-virals; anticoagulants; anticonvulsants; antidepressants; antihistamines; and immunological agents.

Other examples of suitable bioactive agents which may be included in the coating composition include viruses and cells, peptides, polypeptides and proteins, analogs, muteins, and active fragments thereof, such as immunoglobulins, antibodies, cytokines (e.g. lymphokines, monokines, chemokines), blood clotting factors, hemopoietic factors, interleukins (IL-2, IL-3, IL-4, IL-6), interferons (β-IFN, (α-IFN and γ-IFN), erythropoietin, nucleases, tumor necrosis factor, colony stimulating factors (e.g., GCSF, GM-CSF, MCSF), insulin, anti-tumor agents and tumor suppressors, blood proteins, gonadotropins (e.g., FSH, LH, CG, etc.), hormones and hormone analogs (e.g., growth hormone), vaccines (e.g., tumoral, bacterial and viral antigens); somatostatin; antigens; blood coagulation factors; growth factors (e.g., nerve growth factor, insulin-like growth factor); protein inhibitors, protein antagonists, and protein agonists; nucleic acids, such as antisense molecules, DNA and RNA; oligonucleotides; and ribozymes.

A single bioactive agent may be utilized to form the coating composition or, in alternate embodiments, any combination of bioactive agents may be utilized to form the coating composition applied in accordance with the present disclosure.

The amounts of bioabsorbable coating composition to be applied to the suture vary depending upon the specific construction of the suture, the size and the material of this construction. In general, the coating composition applied to an unfilled suture will constitute from about 1.0 to about 3.0 percent by weight of the coated suture, but the amount of coating composition on may range from as little as about 0.5 percent, by weight, to as much as 4.0 percent or higher. For a preferred filled (i.e., containing a storage stabilizing agent) braided suture, amounts of coating composition will generally vary from about 0.5% to 2.0% with as little as 0.2% to as much as 3.0%. As a practical matter and for reasons of economy and general performance, it is generally preferred to apply the minimum amount of coating composition consistent with good surface lubricity and/or knot tie-down characteristics and this level of coating add on is readily determined experimentally for any particular suture.

The described embodiments of the present disclosure are intended to be illustrative rather than restrictive, and are not intended to represent every embodiment of the present invention. Various modifications and variations can be made without departing from the scope of the invention as set forth in the following claims both literally and in equivalents recognized in law.

## Claims

1. A system (1) for coating a suture line (10) comprising:
a coating tube (24) ;
a first guide (13) positioned to orient the suture line (10) for entry into the coating tube (24); and
a second guide (39) positioned to orient the suture line (10) exiting the coating tube,
wherein the coating tube (24) possesses an inlet opening (92) to admit the suture line (10) passing through the first guide (13) and an outlet opening (93) through which the suture line (10) exits the coating tube (24), and the coating tube (24) includes at least one fill opening (56) through which a coating composition is introduced into the coating tube (24) to at least a level sufficient to submerge at least one portion of the suture line (10), **characterised in that**
the coating tube (24) further comprises at each end one coater head (18, 20), each coater head (18, 20) is configured to collect coating composition flowing out of respectively the inlet or outlet opening (92, 93).

2. A system as in claim 1, further comprising:
a return composition tank (32) to collect the coating composition flowing through the inlet and outlet openings.

3. A system as in claim 2, further comprising:
a controller (48) to determine a ratio of at least one coating agent and at least one solvent carrier in the coating composition flowing through the inlet and outlet openings (92, 93); and
a solvent supply means (96) for adding the solvent to the coating composition as a function of the ratio.

4. A system as in claim 3, wherein the solvent supply means (96) supply the solvent to the return composition tank (32) and the coating tube (24).

5. A system as in any one of the preceding claims, further comprising a dryer (6) which dries the suture line (10) at a temperature of from 25° C to 100°C.

6. A system as in any one of the preceding claims arranged so that the coating composition is circulated.

7. A system as in any one of the preceding claims, the at least one coater head (18, 20) disposed at each end of the coating tube (24) comprising a removable reducing tee (80) configured to drain coating composition flowing through the inlet and oulet openings (92, 93).

8. A system as in claim 7, whereby a return adapter (22) is the reducing portion.

9. The system as in any one of the preceding claims, arranged so that the suture line (10) comes into contact only with the coating composition between the first and second guides (13, 39).

10. A method for coating a suture line (10) comprising:
orienting the suture line (10) through a first guide (13) for entry into a coating tube (24), the coating tube (24) possessing an inlet opening (92) to admit the suture line (10) and an outlet opening (93) through which the suture line (10) exits the coating tube (24), the coating tube (24) also possessing at least one fill opening (56) through which a coating composition is introduced into the coating tube (24) to at least a level sufficient to submerge at least one portion of the suture line (10);
submerging the suture line (10) in the coating composition filling the coating tube (24); and
orienting the suture line (10) exiting from the coating tube (24) through a second guide (39);
**characterised in that** the coating tube (24) further includes at least one coater head (18, 20) disposed at each end of the coating tube (24) and the at least one coater head (18, 20) collects coating composition flowing through the inlet and outlet openings (92, 93).

11. A method as in claim 10, further comprising the step of:
collecting the composition flowing through the inlet and outlet openings (92, 93) in a return composition tank (32).

12. A method as in claim 11, further comprising the step of:
determining a ratio of a coating agent and a solvent in the coating composition flowing through the inlet and outlet openings (92, 93); and
adding the solvent to the coating composition as a function of the ratio.

13. A method as in claim 12, wherein the adding the solvent step includes supplying the solvent to the return composition tank (32) and the coating tube (24).

14. A method as in any one of claims 10 to 13, further comprising drying the suture line exiting from the coating tube at a temperature in the range of from 25°C to 100°C.

15. A method as in claim 14, further comprising the steps of :
blowing a gas onto the suture line (10) exiting the coating tube (24) prior to drying; and
cooling the suture line (10) prior to passing the at least one suture line (10) through the second guide (39).

16. A method as in any one of claims 10 to 15, wherein the step of submerging the suture line (10) includes determining the level sufficient to submerge and wet the at least one suture line (10).

17. A method as in any one of claims 10 to 16, comprising circulating the coating composition.

18. A method as in any one of claims 10 to 17, wherein the at least one coater head (18, 20) disposed at each end of the coating tube (24) comprises a reducing tee (80) draining coating composition flowing through the inlet and outlet openings (92, 93).

19. A method as in any one of the preceding claims, wherein the suture line (10) comes into contact only with the coating composition between the first and second guides (92, 93).

## Patentansprüche

1. System (1) zum Beschichten eines chirurgischen Nahtmaterials (10), umfassend:
ein Beschichtungsrohr (24);
eine erste Führung (13), welche angeordnet ist, das chirurgische Nahtmaterial (10) für einen Eintritt in das Beschichtungsrohr (24) auszurichten; und
eine zweite Führung (39), welche angeordnet ist, das chirurgische Nahtmaterial (10), welches das Beschichtungsrohr verlässt, auszurichten,
wobei das Beschichtungsrohr (24) eine Einlaßöffnung (92), um das chirurgische Nahtmaterial (10), welches durch die erste Führung (13) verläuft, zuzuführen, und eine Auslassöffnung (93), durch welche das chirurgische Nahtmaterial (10) das Beschichtungsrohr (24) verlässt, aufweist, und wobei das Beschichtungsrohr (24) mindestens eine Füllöffnung (56) aufweist, durch welche eine Beschichtungszusammensetzung in das Beschichtungsrohr (24) auf mindestens einen Pegel eingeführt wird, welcher ausreichend ist, um zumindest einen Abschnitt des chirurgischen Nahtmaterials (10) einzutauchen, **dadurch gekennzeichnet, dass**
das Beschichtungsrohr (24) ferner an jedem Ende einen Beschichtungskopf (18, 20) umfasst, wobei jeder Beschichtungskopf (18, 20) ausgestaltet ist, Beschichtungszusammensetzung aufzufangen, welche aus der entsprechenden Einlass- oder Auslassöffnung (92, 93) fließt.

2. System nach Anspruch 1, ferner umfassend:
einen Zusammensetzungsrückführungstank (32), um die Beschichtungszusammensetzung zu sammeln, welche durch die Einlass-und Auslassöffnungen (92, 93) fließt.

3. System nach Anspruch 2, ferner umfassend:
eine Steuerung (48), um ein Verhältnis von mindestens einem Beschichtungsmittel und mindestens einem Lösungsmittelträger in der Beschichtungszusammensetzung, welche durch die Einlass- und Auslassöffnungen (92, 93) fließt, festzulegen; und
ein Lösungsmittelzuführungsmittel (96) zum Hinzufügen des Lösungsmittels zu der Beschichtungszusammensetzung als eine Funktion des Verhältnisses.

4. System nach Anspruch 3, wobei das Lösungsmittelzuführungsmittel (96) das Lösungsmittel zu dem Zusammensetzungsrückführungstank (32) und dem Beschichtungsrohr (24) führt.

5. System nach einem der vorhergehenden Ansprüche, ferner umfassend einen Trockner (6), welcher das chirurgische Nahtmaterial (10) bei einer Temperatur von 25°C bis 100°C trocknet.

6. System nach einem der vorhergehenden Ansprüche, welches derart ausgestaltet ist, dass die Beschichtungszusammensetzung in Umlauf gebracht wird.

7. System nach einem der vorhergehenden Ansprüche, wobei der mindestens eine Beschichtungskopf (18, 20), welcher an jedem Ende des Beschiehtungsrohrs (24) angeordnet ist, eine entfernbare Verringerungsabzweigung (80) umfasst, welche ausgestaltet ist, Beschichtungszusammensetzung, welche durch die Einlass- und Auslassöffnungen (92, 93) fließt, ablaufen zu lassen.

8. System nach Anspruch 7, wobei ein Rückführungsadapter (22) der Verringerungsabschnitt ist.

9. System nach einen der vorhergehenden Ansprüche, welches derart ausgestaltet ist, dass das chirurgische Nahtmaterial (10) zwischen der ersten und zweiten Führung (13, 39) nur mit der Beschichtungszusammensetzung in Kontakt kommt.

10. Verfahren zum Beschichten eines chirurgischen Nahtmaterials (10), umfassend:
Ausrichten des chirurgischen Nahtmaterials (10) durch eine erste Führung (13) für einen Eintritt in ein Beschichtungsrohr (24), wobei das Beschichtungsrohr (24) eine Einlassöffnung (92), um das chirurgische Nahtmaterial (10) zuzuführen,
und eine Auslassöffnung (93), durch welche das chirurgische Nahtmaterial (10) das Beschichtungsrohr (24) verlässt, aufweist, wobei das Beschichtungsrohr (24) ferner mindestens eine Füllöffnung (56) aufweist, durch welche eine Beschichtungszusammensetzung in das Beschichtungsrohr (24) auf mindestens einen Pegel eingeführt wird, welcher ausreichend ist,
um zumindest einen Abschnitt des chirurgischen Nahtmaterials (10) einzutauchen;
Eintauchen des chirurgischen Nahtmaterials (10) in die Beschichtungszusammensetzung, welche das Beschichtungsrohr (24) füllt; und
Ausrichten des chirurgischen Nahtmaterials (10), welches aus dem Beschichtungsrohr (24) austritt, durch eine zweite Führung (39) ;
**dadurch gekennzeichnet, dass** Beschichtungsrohr (24) ferner mindestens einen Beschichtungskopf (18, 20) aufweist, welcher an jedem Ende des Beschichtungsrchrs (24) angeordnet ist, und
dass der mindestens eine Beschichtungskopf (18, 20) Beschichtungszusammensetzung aufsammelt, welche durch die Einlass-und Auslassöffnungen (92, 93) fließt.

11. Verfahren nach Anspruch 10, ferner umfassend den Schritt:
Aufsammeln der Zusammensetzung, welche durch die Einlass-und Auslassöffnungen (92, 93) fließt, in einem Zusammensetzungsrückführungstank (32).

12. Verfahren nach Anspruch 11, ferner umfassend den Schritt:
Festlegen eines Verhältnisses von einem Beschichtungsmittel und einem Lösungsmittel in der Beschichtungszusammensetzung, welche durch die Einlass- und Auslassöffnungen (92, 93) fließt; und
Hinzufügen des Lösungsmittels zu der Beschichtungszusammensetzung als eine Funktion des Verhältnisses.

13. Verfahren nach Anspruch 12, wobei der Schritt des Hinzufügens des Lösungsmittels ein Zuführen des Lösungsmittels zu dem Zusammensetzungsrückführungstank (32) und dem Beschichtungsrohr (24) aufweist.

14. Verfahren nach einem der Ansprüche 1-13, ferner umfassend ein Trocknen des chirurgischen Nahtmaterials, welches das Beschichtungsrohr verlässt, bei einer Temperatur in einem Bereich von 25°C bis 100°C.

15. Verfahren nach Anspruch 14, ferner umfassend die Schritte:
Blasen eines Gases auf das chirurgische Nahtmaterial (10), welches das Beschichtungsrohr (24) verlässt, vor dem Trocknen; und
Kühlen des chirurgischen Nahtmaterials (10) bevor das mindestens eine chirurgische Nahtmaterial (10) durch die zweite Führung (39) verläuft.

16. Verfahren nach einem der Ansprüche 10-15, wobei der Schritt des Eintauchens des chirurgischen Nahtmaterials (10) ein Bestimmen des Pegels, welcher zum Eintauchen und Befeuchten des mindestens einen chirurgischen Nahtmaterials (10) ausreichend ist, aufweist.

17. Verfahren nach einem der Ansprüche 10-16, umfassend ein in Umlauf Bringen der Beschichtungszusammensetzung.

18. Verfahren nach einem den Ansprüche 10-17, wobei der mindestens eine Besahichtungskopf (18, 20), welcher an jedem Ende des Beschichtungsrohrs (24) angeordnet ist, eine Verringerungsabzweigung (80) umfasst, welche Beschichtungszusammensetzung, welche durch die Einlass- und Auslassöffnungen (92, 93) fließt, ablaufen lässt.

19. Verfahren nach einem der vorhergehenden Ansprüche, wobei das chirurgische Nahtmaterial (10) zwischen der ersten und der zweiten Führung (92, 93) nur mit der Beschichtungszusammensetzung in Kontakt kommt.

## Revendications

1. Système (1) pour revêtir une ligne de fil de suture (10) comprenant :
un tube d'enduction (24) ;
un premier guide (13) positionné de manière à orienter la ligne de fil de suture (10) pour la faire entrer dans le tube d'enduction (24) ; et
un deuxième guide (39) positionné de manière à orienter la ligne de fil de suture (10) sortant du tube d'enduction,
dans lequel le tube d'enduction (24) comporte une ouverture d'entrée (92) pour recevoir la ligne de fil de suture (10) passant dans le premier guide (13) et une ouverture de sortie (93) par laquelle la ligne de fil de suture (10) sort du tube d'enduction (24), et le tube d'enduction (24) comprend au moins une ouverture de remplissage (56) par laquelle une composition de revêtement est introduite dans le tube d'enduction (24) au moins jusqu'à un niveau suffisant pour immerger au moins une partie de la ligne de fil de suture (10),
**caractérisé en ce que** le tube d'enduction (24) comprend en outre à chaque extrémité une tête d'enduction (18, 20), chaque tête d'enduction (18, 20) étant configurée pour collecter la composition de revêtement qui sort respectivement par l'ouverture d'entrée ou l'ouverture de sortie (92, 93).

2. Système selon la revendication 1, comprenant en outre un réservoir de composition récupérée (32) pour collecter la composition de revêtement qui passe par les ouvertures d'entrée et de sortie.

3. Système selon la revendication 2, comprenant en outre :
un dispositif de commande (48) pour déterminer un rapport d'au moins un agent de revêtement et au moins un support de solvant dans la composition de revêtement qui passe par les ouvertures d'entrée et de sortie (92, 93) ; et
un moyen d'alimentation en solvant (96) pour ajouter le solvant à la composition de revêtement en fonction de ce rapport.

4. Système selon la revendication 3, dans lequel le moyen d'alimentation en solvant (96) envoie le solvant dans le réservoir de composition récupérée (32) et dans le tube d'enduction (24).

5. Système selon l'une quelconque des revendications précédentes, comprenant en outre un séchoir (6) qui sèche la ligne de fil de suture (10) à une température de 25 °C à 100 °C.

6. Système selon l'une quelconque des revendications précédentes, agencé de telle manière que la composition de revêtement est mise en circulation.

7. Système selon l'une quelconque des revendications précédentes, dans lequel ladite au moins une tête d'enduction (18, 20) disposée à chaque extrémité du tube d'enduction (24) comprend un té de réduction amovible (80) configuré pour évacuer la composition de revêtement qui passe par les ouvertures d'entrée et de sortie (92, 93).

8. Système selon la revendication 7, dans lequel un adaptateur de retour (22) constitue la partie de réduction.

9. Système selon l'une quelconque des revendications précédentes, agencé de telle manière que la ligne de fil de suture (10) vient en contact uniquement avec la composition de revêtement entre les premier et deuxième guides (13, 39).

10. Procédé pour revêtir une ligne de fil de suture (10) comprenant les étapes suivantes :
orienter la ligne de fil de suture (10) dans un premier guide (13) pour la faire entrer dans le tube d'enduction (24), le tube d'enduction (24) comportant une ouverture d'entrée (92) pour recevoir la ligne de fil de suture (10) et une ouverture de sortie (93) par laquelle la ligne de fil de suture (10) sort du tube d'enduction (24), le tube d'enduction (24) comprenant de plus au moins une ouverture de remplissage (56) par laquelle une composition de revêtement est introduite dans le tube d'enduction (24) au moins jusqu'à un niveau suffisant pour immerger au moins une partie de la ligne de fil de suture (10) ;
immerger la ligne de fil de suture (10) dans la composition de revêtement qui remplit le tube d'enduction (24) ; et
orienter la ligne de fil de suture (10) sortant du tube d'enduction (24) dans un deuxième guide (39) ;
**caractérisé en ce que** le tube d'enduction (24) comprend en outre au moins une tête d'enduction (18, 20) placée à chaque extrémité du tube d'enduction (24) et ladite au moins une tête d'enduction (18, 20) collecte la composition de revêtement qui passe par les ouvertures d'entrée et de sortie (92, 93).

11. Procédé selon la revendication 10, comprenant en outre l'étape consistant à collecter la composition qui passe par les ouvertures d'entrée et de sortie (92, 93) dans un réservoir de composition récupérée (32).

12. Procédé selon la revendication 11, comprenant en outre les étapes suivantes :
déterminer un rapport d'un agent de revêtement et d'un solvant dans la composition de revêtement qui passe par les ouvertures d'entrée et de sortie (92, 93) ; et
ajouter le solvant à la composition de revêtement en fonction de ce rapport.

13. Procédé selon la revendication 12, dans lequel l'étape d'ajout du solvant comprend le fait d'envoyer le solvant dans le réservoir de composition récupérée (32) et dans le tube d'enduction (24).

14. Procédé selon l'une quelconque des revendications 10 à 13, comprenant en outre le fait de sécher la ligne de fil de suture sortant du tube d'enduction à une température comprise dans l'intervalle de 25 °C à 100 °C.

15. Procédé selon la revendication 14, comprenant en outre les étapes suivantes :
projeter un gaz sur la ligne de fil de suture (10) sortant du tube d'enduction (24) avant le séchage ; et
refroidir la ligne de fil de suture (10) avant de faire passer ladite au moins une ligne de fil de suture (10) dans le deuxième guide (39).

16. Procédé selon l'une quelconque des revendications 10 à 15, dans lequel l'étape d'immersion de la ligne de fil de suture (10) comprend la détermination du niveau suffisant pour immerger et mouiller ladite au moins une ligne de fil de suture (10).

17. Procédé selon l'une quelconque des revendications 10 à 16, comprenant le fait de faire circuler la composition de revêtement.

18. Procédé selon l'une quelconque des revendications 10 à 17, dans lequel ladite au moins une tête d'enduction (18, 20) disposée à chaque extrémité du tube d'enduction (24) comprend un té de réduction (80) qui évacue la composition de revêtement qui passe par les ouvertures d'entrée et de sortie (92, 93).

19. Procédé selon l'une quelconque des revendications précédentes, dans lequel la ligne de fil de suture (10) vient en contact uniquement avec la composition de revêtement entre les premier et deuxième guides (13, 39).
